# EUROPEAN PATENT SPECIFICATION

(11) **EP 2 981 537 B1**
(45) Date of publication and mention of the grant of the patent: **10.05.2017**
(21) Application number: 13861489.6
(22) Date of filing: 14.03.2013
(51) Int. Cl.: C07D 487/04, C07C 279/14, C07C 279/18

(54) **PROCESS FOR MAKING ANAGRELIDE**
VERFAHREN ZUR HERSTELLUNG VON ANAGRELID
PROCÉDÉ DE PRÉPARATION D'ANAGRÉLIDE

(43) Date of publication of application: 10.02.2016
(73) Proprietor: Synthon B.V., 6545 CM Nijmegen (NL)
(72) Inventor: GIELING, Reinerus, Gerardus, 6545CM Nijmegen (NL); LINDEN VAN DER, Johannes, Bastiaan, 6545 CM Nijmegen (NL); VERKERK, Pascal, Renart, 6545 CM Nijmegen (NL); MELSA, Petr, 67817 Blansko (CZ)
(74) Representative: Mendivil Gil, Maria Dolores
(86) International application number: PCT/EP2013/055231
(87) International publication number: WO 2014/139572

(56) References cited:
- EP-A1- 0 514 917
- US-A- 4 146 718
- US-A- 4 208 521
- HITOSHI YAMAGUCHI, FUMIYOSHI ISHIKAWA: "Synthesis and reactions of 2-chloro-3,4-dihydrothienopyrimidines and -quinazolines", J. HETEROCYCLIC CHEM., vol. 18, 1980, pages 67-70, XP002726341,

## Description

The present invention relates to an improved process for making the pharmaceutically active compound anagrelide.

Anagrelide, chemically 6,7-dichloro-1,2,3,5-tetrahydroimidazo[2,1b]quinazolin-2-one of formula (1) 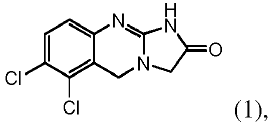 is a compound useful for the treatment of (essential) thrombocythaemia.

The commercially marketed products Xagrid® and Agrylin® contain the hydrochloride monohydrate salt of the title compound (1). Anagrelide hydrochloride monohydrate is an off-white powder that is very slightly soluble in water.

Anagrelide was first disclosed in US 3,932,407 (i.e., compound 47). Said document also disclosed a process for making anagrelide, comprising chlorinating the 7-deschloroanagrelide analogue compound of formula (1A) depicted below (cf. Example 25). Said compound of formula (1A) was prepared by a process comprising the steps of
a) reducing the nitrobenzyl glycinate compound of formula (2A) with hydrogen in the presence of a noble metal catalyst, particularly palladium on carbon, yielding the aminobenzyl glycinate compound of formula (3A), and
b) reacting the compound of formula (3A) with cyanogen bromide in an inert solvent, e.g. an alcohol, followed by treatment with alkalinized water.

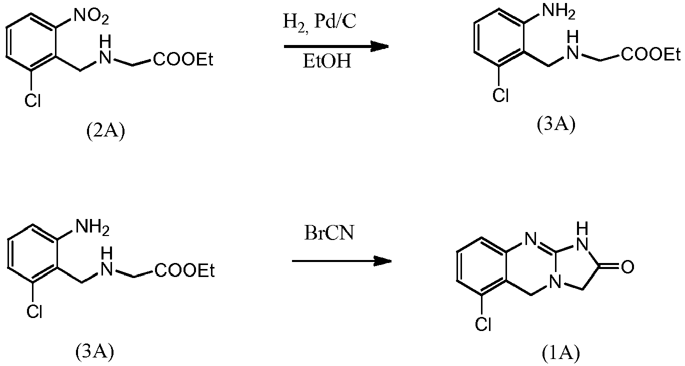

An improved process for making the compound of formula (1A) was disclosed in US 3,932,467.

US 4,146,718 disclosed that the same process can be used for making anagrelide, in particular by reaction of the compound of formula (3), wherein R is an alkyl group, e.g. the compound of formula (3B), with cyanogen bromide.

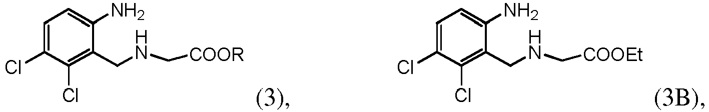

Furthermore, it was disclosed that much higher yields were obtained by reacting the alkyl aminobenzyl glycinate compound of formula (3) with cyanogen halide in an aprotic solvent (e.g. in a hydrocarbon, halohydrocarbon, or ether), isolating the formed iminoquinazoline of formula (4), wherein R is an alkyl group and X is a chloro, bromo or iodo, e.g., the compound of formula (4B), 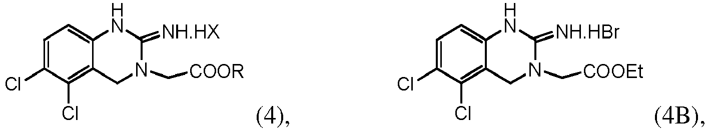 and reacting the compound of formula (4) in a solvent with a base, preferably an organic base, typically triethylamine. Essentially, the same process was disclosed in WO 2009/087673, WO 2010/070318, WO 2012/052781, and WO 2005/080398 (using sodium carbonate instead of an organic base in the last two cases).

In US 4,208,521, an alternative process for making anagrelide and its derivatives was disclosed in which the compound of formula (3) reacts in an inert solvent with a compound of formula (6),

H₂N-C(=NH)X (6),

wherein X is a leaving group selected from amino, alkyl/arylthio or a nitrogen-attached N-heterocycle. The reported yield for anagrelide after 24 hours of reflux was 40%, when the compound of formula (6) was 3,5-dimethyl-pyrazole-1-carboxamidine nitrate (the most suitable example of the compound of formula (6)).

Another alternative process has been disclosed in US 5,391,737. The process comprises a thermal cyclization of a cyanoimine compound, e.g., of formula (7), in acidic medium, e.g. in a highly boiling ether solvent in the presence of concentrated aqueous HCl. The anagrelide may be isolated after neutralization as a free base or, without neutralization, directly as a hydrated hydrochloride.

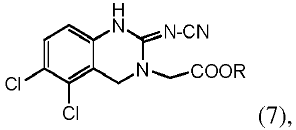

The compound of formula (7) can be prepared from the compound of formula (3) by reaction with, e.g., diphenyl-N-cyanoimidocarbonate.

The starting compound of formula (3) in any of the above-mentioned processes may be prepared by various ways, e.g.:
a) by reduction of the corresponding nitro-compound of formula (2), wherein R is an alkyl group, e.g. the compound of formula (2B), by stannous chloride as described in US 4,146,718 or by catalytic hydrogenation as suggested (but not exemplified) in WO 2010/070318: 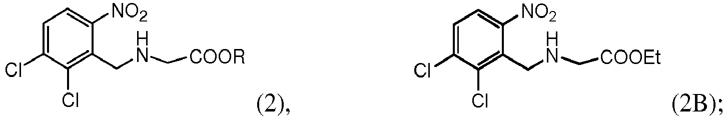 or
b] by condensation of the aminobenzyl chloride of formula (5), with a glycine ester, e.g. glycine ethylester (cf. US 4,146,718): 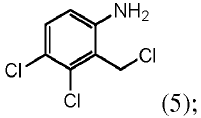 or
c] by condensation of the aminobenzyl amine of formula (5A), with a bromoacetate (cf. WO 2005/080398).

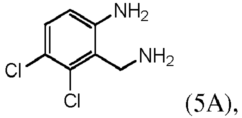

While in general, anagrelide is produced as the free base by the above procedures, it may be converted into a suitable acid addition salt by treatment with a pharmaceutically acceptable acid. To make the pharmaceutically desirable anagrelide hydrochloride monohydrate, anagrelide free base is heated in methanol with concentrated aqueous HCl, and the obtained solution is cooled and treated with ether as an antisolvent (cf. US 4,146,718). Initially, a product with half the theoretical water content is obtained. The desired water content is obtained only by standing in a high humidity chamber. In another version, a suspension of anagrelide free base in an alcohol is heated to reflux, water is added, the mixture is acidified with hydrochloric acid, refluxed and rapidly cooled (cf. US 2010/0305318). In yet another version, a mixture of anagrelide and 5-6 N aqueous HCl in methanol is heated to reflux, the hot solution is filtered and the filtrate is cooled (cf. WO 2005/080398, no water content reported).

While processes of making anagrelide and acid addition salts are known in the art, there is still a need for improvement in the matter.

### BRIEF DESCRIPTION OF THE PRESENT INVENTION

The object of the present invention is a process for the manufacture of anagrelide from a compound of general formula (3) with improved yield and purity. The present invention provides an alternative process for the manufacture of anagrelide, which does not utilize extremely toxic cyanogen halide.

In a first aspect, the present invention relates to a process for making anagrelide of formula (1) 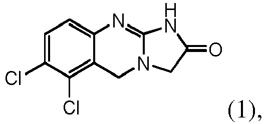 or an acid addition salt thereof, including any hydrated or solvated form thereof, comprising reacting a compound of formula (3), or an acid addition salt thereof, 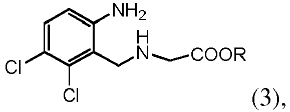 wherein R is a C1-C4 alkyl group, with chloroformamidine hydrochloride of formula (8) 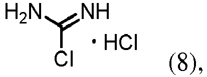 in an inert solvent, followed by treatment of the reaction mixture with a base.

In one embodiment of the present invention, the group R is an ethyl group.

In another embodiment, the inert solvent is selected from the group consisting of C5-C10 aliphatic hydrocarbons, C5-C10 alicyclic hydrocarbons, C6-C10 aromatic hydrocarbons, C1-C6 chlorinated hydrocarbons, C5-C10 aliphatic or cyclic ethers, C3-C10 aliphatic esters, dimethylformamide, dimethylsulfoxide, acetonitrile, acetic acid, or any combination thereof.

In a further embodiment, the compound of formula (3) reacts with the compound of formula (8) at a temperature of from 50°C to reflux temperature.

In a yet further embodiment, the product of the reaction between the compounds of formulas (3) and (8) is isolated from the reaction mixture before treatment with the base.

Advantageously, the base is an organic amine or a metal carbonate.

In a specific embodiment, anagrelide is isolated from the reaction mixture as a free base after treatment with the base.

In another embodiment, the anagrelide free base is converted into anagrelide hydrochloride, and is isolated as a solid material, optionally in any solvated or hydrated form.

### DETAILED DESCRIPTION OF THE INVENTION

The present invention relates to a novel advantageous process for making anagrelide of formula (1) as shown above or an acid addition salt thereof. In particular, the process may be used for making anagrelide hydrochloride monohydrate, which is the active substance in currently marketed pharmaceutical compositions comprising anagrelide.

The process according to the present invention starts from the compound of general formula (3), which can be obtained by various processes known in the art, which processes are briefly overviewed above. Furthermore, some compounds within the general formula (3), in particular the ethyl ester compound of formula (3B), are now commercially available.

In comparison with the first general process of the prior art outlined above, the process of the present invention does not employ an extremely toxic cyanogen halide, such as cyanogen bromide. The process of the present invention provides the desired anagrelide in a single-step procedure, without the explicit need of making and isolating the intermediate compound of formula (4). In comparison with other prior art procedures, characterised by direct conversion of the compound (3) to anagrelide without making and isolating an intermediate, the process of the present invention provides anagrelide in much higher yields. Notably, the process disclosed in US 4,146,718 provided anagrelide in only 31% yield; that of US 4,208,521 provided anagrelide in only 40% yield, and required long reaction times. To the contrary, anagrelide can be obtained by the process of the present invention in a yield of at least 65%, in shorter reaction times.

The process of the present invention is based on reacting a compound of formula (3), or an acid addition salt thereof, 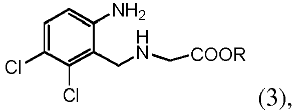 wherein R is a C1-C4 alkyl group, with chloroformamidine hydrochloride of formula (8) 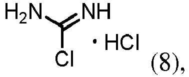 in an inert solvent, followed by treatment of the reaction mixture with a base.

Chloroformamidine hydrochloride is a known compound. It can be purchased commercially or it may be prepared by processes known in the art.

The preferred compound of general formula (3) is the ethyl ester of formula (3B) above. This compound is commercially available.

A suitable acid addition salt of any of the compounds of formula (3) is, e.g., the corresponding hydrochloride salt.

In the process of the present invention, the molar amount between the compounds of formulas (3) and (8) typically is from 1 : 1 to 1 : 2, advantageously from 1 : 1.2 to 1: 1.7, and more advantageously about 1 : 1.5.

The type of solvent to be used in accordance with the present invention, in which the reaction between the compounds of formulas (3) and (8) takes place, is essentially not limited to any particular type of solvent, with the proviso that the solvent is inert with respect to the reagents and the reaction product. In an advantageous embodiment, the solvent is an aprotic solvent. It may be, e.g., a hydrocarbon, such as a C5-C10 aliphatic hydrocarbon, a C5-C10 alicyclic hydrocarbon or a C6-C10 aromatic hydrocarbon, a halogenated hydrocarbon, such as a C1-C6 chlorinated hydrocarbon, a C5-C10 aliphatic or cyclic ether, a C3-C10 aliphatic ester, a dipolar aprotic solvent such as dimethylformamide, dimethylsulfoxide or acetonitrile, or acetic acid, or any combination thereof. Suitable examples of the various groups of solvents mentioned above include n-hexane, cyclohexane, toluene, dichloroethane, tetrahydrofuran, isopropyl acetate, acetonitrile, and acetic acid. The solvents may also be used in any combination. Polar solvents are advantageous, because of a generally shorter reaction time. Water-miscible solvents or solvent combinations are preferred, because of easier work-up of the reaction mixture. A mixture of acetonitrile or tetrahydrofuran with acetic acid is the most preferred solvent combination/mixture.

The reaction temperature used in the process of the present invention is, in general, higher than 40°C, preferably 50°C or higher, and includes temperatures up to the boiling point of the solvent or solvent mixture (i.e., reflux temperature).

The reaction can be monitored by any suitable analytical method, e.g. HPLC, and may be terminated after reaching the desired conversion of the starting material of formula (3). The primary product of the reaction is a guanidine compound, either of formula (9A) or formula (9B), or a mixture of both compounds.

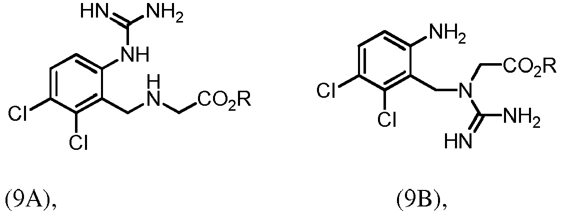

In the above formulae, R has the same meaning as in the compound of formula (3) and preferably is an ethyl group. The formulae (9A) and (9B) also encompass any acid addition salts thereof, e.g. hydrochloride salts. In accordance with a preferred embodiment of the present invention, this primary product needs not to be isolated from the reaction mixture, but a process variant comprising isolation of the above primary product(s) is not excluded and forms part of the present invention.

In accordance with the present invention, the reaction mixture is subsequently treated with a base. In one embodiment, the base is a primary, secondary or tertiary organic amine. The nature of the amine is not particularly limited, preferred are commercially available liquid amines of 4-10 carbon atoms such as triethyl amine, pyridine, diisopropylethylamine, etc. In another embodiment, the base is a metal carbonate, preferably sodium or potassium carbonate or hydrogen carbonate.

The amount of the base is such, that it is sufficient to make the reaction mixture alkaline. Preferably, at least 1 molar equivalent of base is used, with respect to the compound of formula (8).

The base may be charged as such or in a suitable liquid carrier. It may be charged in one portion or gradually.

The solvent for the reaction with the base may be the same as used for the reaction between the compounds of formulas (3) and (8). Alternatively, the original solvent may be removed and replaced with another one before treatment with the base. In such an arrangement, even protic solvents, such as aliphatic alcohols, can be employed for the reaction with the base.

The reaction temperature for the reaction with the base may be any temperature from 0°C to reflux temperature.

The reaction with the base can be monitored by any suitable analytical method, e.g. HPLC.

The primary product of the reaction with the base is the compound of formula (4) or, rather, the free base thereof of formula (4A) below. It should be borne in mind that the structure of the compound (4) can be depicted in two tautomeric forms as shown below

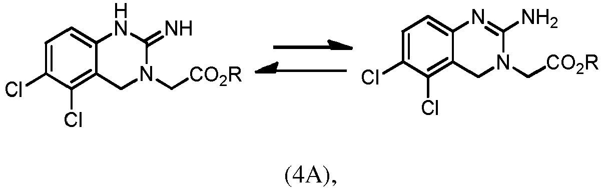

In general, the product is relatively unstable under the reaction conditions disclosed above, particularly in alkaline environment, and easily undergoes cyclization into the desired anagrelide of formula (1). However, it is not excluded that the reaction conditions can be so adjusted that the product of formula (4A) can be obtained and isolated. Such modification is also within the scope of the present invention.

Thus, after the treatment with a base under the conditions disclosed above, anagrelide of formula (1) is formed. It may be isolated from the reaction mixture by common means, e.g. by filtration or centrifugation. As the process of the present invention also produces other products such as chloride salts, it is advantageous that such side products are removed from the desired anagrelide product. In one embodiment, the reaction mixture is treated with water, in which these side products are soluble, and anagrelide is filtered off after such treatment. In another embodiment, the side products are separated from the solid anagrelide by washing with water or by stirring the separated anagrelide in water.

The separated/isolated anagrelide may finally be dried and milled, if desired.

By the above process, anagrelide is obtained as a free base. In a next step, it may be converted into a suitable acid addition salt, e.g. a hydrochloride salt, by contacting the free base with the corresponding acid, which acid addition salt is isolated in a suitable solid state form, which includes any hydrated or solvated form. Both anagrelide as isolated from the invention process, as well as a reaction mixture comprising anagrelide obtained by the process according to the present invention, may be used as the starting material for this conversion into an acid addition salt.

In particular, anagrelide obtained by the process of the present invention is converted into anagrelide hydrochloride monohydrate following procedures known in the art, some of which procedures were briefly overviewed above.

The invention will be further illustrated by way of the following examples.

### EXAMPLES

### Example 1

A 11 three-neck round-bottom flask equipped with a mechanical stirrer, a condenser, and a thermocouple was charged with ethyl 2-(6-amino-2,3-dichlorobenzylamino)acetate of formula (3B) (20.00 g, 72.0 mmol), chloroformamidine hydrochloride (12.45 g, 108 mmol) and acetonitrile (240 ml) under argon, and the suspension was heated to 40°C. Then, acetic acid (21 ml, 363 mmol) was added and the suspension was heated to 78°C. After 9.5 hours, the starting compound (3B) was fully consumed as checked by HPLC. Then N,N-diisopropylethylamine (44 ml, 252 mmol) was added drop-wise over approx. 15 minutes, while the heating bath was removed to maintain a temperature of 75-78°C. At the end of addition, acetonitrile (60 ml) was added to dilute the reaction mixture. The resulting suspension was stirred for an additional 1 hour, and was then cooled to 23°C, and stirred for an additional 2 hours. Then, the suspension was filtered, washed with water (500 ml), ethanol (100 ml), and dried (30°C, 100 mbar, N₂ bleed, 14.5 hours) to give 12.99 g of anagrelide as a pale beige solid (70% of theory) in 99.47% purity (HPLC, external standard).

### Example 2

A 11 three-neck round-bottom flask equipped with a mechanical stirrer, a condenser, and a thermocouple was charged with ethyl 2-(6-amino-2,3-dichlorobenzylamino)acetate of formula (3B) (20.00 g, 72.0 mmol), chloroformamidine hydrochloride (12.44 g, 108 mmol) and acetonitrile (120 ml) under argon, and the suspension was heated to 40°C. Then, acetic acid (21 ml, 363 mmol) was added and the suspension was heated to reflux (90°C in oil bath). After 5 hours, the compound of formula (3B) was fully consumed as checked by HPLC. The suspension was diluted with acetonitrile (60 ml), and then N,N-diisopropylethylamine (44 ml, 252 mmol) was added drop-wise over approx. 3 minutes, and the suspension was stirred for an additional 75 minutes. Then, the reaction mixture was cooled to 50°C and water (150 ml) was added. Stirring at 50°C was continued for 1 hour. Then, the suspension was filtered, washed with water (30 ml), ethanol (50 ml), and methyl *t*-butyl ether (50 ml), and dried (23°C, 100 mbar, N₂ bleed, 18 hours) to give 13.48 g of anagrelide as a pale beige solid (73% of theory) in 99.53% purity (HPLC, external standard)).

### Example 3

A 100 ml round-bottom flask equipped with a mechanical stirrer and a condenser was charged with ethyl 2-(6-amino-2,3-dichlorobenzylamino)acetate of formula (3B) (2.50 g, 8.99 mmol) and tetrahydrofuran (30 ml) under argon to give a yellow solution. Then, chloroformamidine hydrochloride (1.55 g, 13.49 mmol) was added, and the suspension was heated to 50°C (in oil bath). Then, acetic acid (2.60 ml, 45.0 mmol) was added, the reaction mixture was heated to 80°C (in oil bath), and stirred for 11 hours to complete the consumption of the compound (3B). Then, N,N-diisopropylethylamine (5.5 ml, 31.5 mmol) was added drop-wise during approx. 3 min to the reaction mixture, and stirring was continued for 1.25 hours. The reaction mixture was cooled to 50°C (in oil bath) and water (40 ml) was added; stirring was continued for 1 hour. The product was filtered, washed with water (12.5 ml), ethanol (12.5 ml), and methyl *t*-butyl ether (25 ml), and dried (50°C, 100 mbar, N₂ bleed, 2 hours) to give 1.78 g of anagrelide as a white solid (76% of theory) in 98.81% purity (HPLC, external standard).

### Example 4

A 500 ml round-bottom flask equipped with a mechanical stirrer and a condenser was charged with ethyl 2-(6-amino-2,3-dichlorobenzylamino)acetate of formula (3B) (10.00 g, 36.0 mmol) and acetonitrile (250 ml) under argon to give a yellow solution. Then, chloroformamidine hydrochloride (6.20 g, 54.0 mmol) was added, and the suspension was stirred at ambient temperature (22-25°C) for 15 minutes. Then, the reaction mixture was heated gradually to 90°C. The reaction mixture was stirred for 19 hours until complete consumption of the compound of formula (3B). Then, N,N-diisopropylethylamine (18.9 ml, 108 mmol) was added to the reaction mixture, while the heating bath was removed. The suspension was stirred for an additional 1 hour. Then, the product was filtered, washed with water (50 ml), ethanol (20 ml), and methyl *t*-butyl ether (30 ml), and the product was dried (40°C, 100 mbar, N₂ bleed, 19 hours) to give 6.34 g of anagrelide as a solid (66% of theory) in 95.9% purity (HPLC, external standard).

### Example 5

In a 250 ml round-bottom flask ethyl 2-(6-amino-2,3-dichlorobenzylamino)acetate of formula (3B) (5.00 g, 18.04 mmol) was stirred in toluene (125 ml) to give a yellow solution. Chloroformamidine hydrochloride (3.11 g, 27.1 mmol) was added, and the mixture was heated to 110°C. Stirring was continued at 110°C for 2.5 hours.

The reaction mixture was concentrated almost to dryness, and was then diluted with ethanol (100 ml). A yellow solution was obtained after gently heating to 60°C. The solution was then made basic with triethylamine (5 ml, 35.9 mmol). A precipitate formed. After stirring for an additional hour at room temperature, the solid was isolated by filtration and washed with water (2x25 ml), ethanol (15 ml), and diethyl ether (25 ml).

The solid was dried on the air, giving anagrelide (3.05 g. 11.91 mmol, 66.0% yield) with a purity of 97+% as an off-white solid.

### Example 6

In a 100 ml round-bottom flask anagrelide (0.50 g, 1.952 mmol) was stirred in DMSO (4 ml) to give a white suspension. The mixture was heated on an oil bath. At 50°C, concentrated hydrochloric acid (0.22 ml, 2.48 mmol) was added. An almost clear solution was obtained after further heating. Ethanol (9 ml) was added, and the clear yellow/orange solution was allowed to cool to room temperature. At 40°C (internal temperature), the solution was seeded with anagrelide hydrochloride crystals. A white suspension was obtained. The suspension was filtered over a glass filter, and the crystalline solid was washed with ethanol, and dried on the air, giving anagrelide hydrochloride (255 mg, 0.863 mmol, 44.2 % yield) as a white crystalline solid.

## Claims

1. A process for making anagrelide of formula (1), 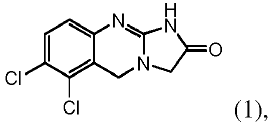 or an acid addition salt thereof, including any hydrated or solvated form thereof, comprising reacting a compound of formula (3), or an acid addition salt thereof, 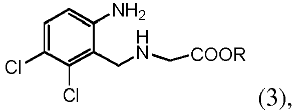 wherein R is a C1-C4 alkyl group, with chloroformamidine hydrochloride of formula (8), 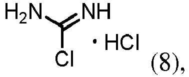 in an inert solvent, followed by treatment of the reaction mixture with a base.

2. The process according to claim 1, wherein R is an ethyl group.

3. The process according to claim 1 or 2, wherein the inert solvent is selected from the group consisting of C5-C10 aliphatic hydrocarbons, C5-C10 alicyclic hydrocarbons, C6-C10 aromatic hydrocarbons, C1-C6 chlorinated hydrocarbons, C5-C10 aliphatic or cyclic ethers, C3-C10 aliphatic esters, dimethylformamide, dimethylsulfoxide, acetonitrile, acetic acid, or any combination thereof.

4. The process according to any one of claims 1-3, wherein the reaction temperature is of from 50°C to reflux temperature.

5. The process according to any one of claims 1-4, wherein the product of the reaction between the compounds of formulas (3) and (8) is isolated from the reaction mixture before treatment with the base.

6. The process according to any one of claims 1-5, wherein the base is an organic amine or a metal carbonate.

7. The process according to any one of claims 1-6, wherein anagrelide is isolated from the reaction mixture as a free base after treatment with the base.

8. The process according to claim 7, wherein the anagrelide free base is converted into anagrelide hydrochloride, and is isolated as a solid material, optionally in any solvated or hydrated form.

9. A compound of formula (9A) or (9B), or an acid addition salt thereof, 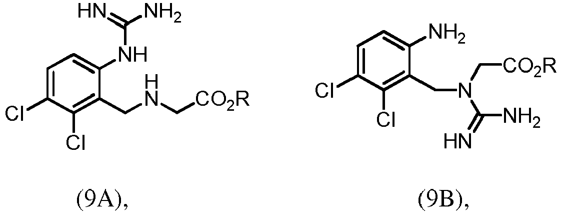 wherein R is a C1-C4 alkyl group, preferably an ethyl group, or a mixture thereof.

## Patentansprüche

1. Verfahren zum Herstellen von Anagrelid der Formel (1), 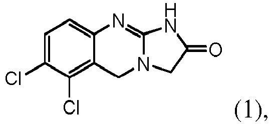 oder eines Säureadditionssalzes davon, einschließlich einer beliebigen hydratisierten oder solvatisierten Form davon,
umfassend Umsetzen einer Verbindung der Formel (3), oder eines Säureadditionssalzes davon, 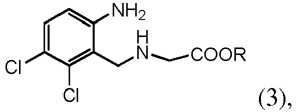 wobei R eine C1-C4-Alkylgruppe ist, mit Chlorformamidinhydrochlorid der Formel (8), 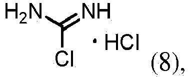 in einem inerten Lösungsmittel, gefolgt von Behandlung des Reaktionsgemisches mit einer Base.

2. Verfahren nach Anspruch 1, wobei R eine Ethylgruppe ist.

3. Verfahren nach Anspruch 1 oder 2, wobei das inerte Lösungsmittel aus der Gruppe bestehend aus C5-C10-aliphatischen Kohlenwasserstoffen, C5-C10-alicyclischen Kohlenwasserstoffen, C6-C10-aromatischen Kohlenwasserstoffen, C1-C6-chlorierten Kohlenwasserstoffen, C5-C10aliphatischen oder cyclischen Ethern, C3-C10-aliphatischen Estern, Dimethylformamid, Dimethylsulfoxid, Acetonitril, Essigsäure, oder einer beliebigen Kombination davon ausgewählt ist.

4. Verfahren nach einem beliebigen der Ansprüche 1-3, wobei die Reaktionstemperatur von 50°C bis zur Rückflusstempe ratur beträgt.

5. Verfahren nach einem beliebigen der Ansprüche 1-4, wobei das Produkt der Reaktion zwischen den Verbindungen der Formeln (3) und (8) aus dem Reaktionsgemisch vor Behandlung mit der Base isoliert wird.

6. Verfahren nach einem beliebigen der Ansprüche 1-5, wobei die Base ein organisches Amin oder ein Metallcarbonat ist.

7. Verfahren nach einem beliebigen der Ansprüche 1-6, wobei Anagrelid aus dem Reaktionsgemisch als eine freie Base nach Behandlung mit der Base isoliert wird.

8. Verfahren nach Anspruch 7, wobei die freie Base des Anagrelid zu Anagrelidhydrochlorid umgewandelt wird und als ein festes Material isoliert wird, gegebenenfalls in einer beliebigen solvatisierten oder hydratisierten Form.

9. Verbindung der Formel (9A) oder (9B), oder ein Säureadditionssalz davon, 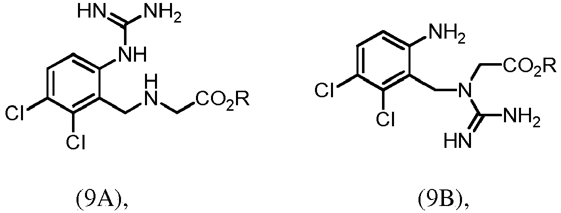 wobei R eine C1-C4-Alkylgruppe, bevorzugt eine Ethylgruppe, oder ein Gemisch davon ist.

## Revendications

1. Un procédé de fabrication d'anagrélide de formule (1), 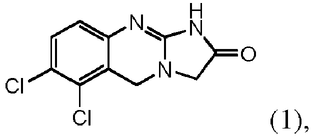 ou un sel d'addition d'acide en dérivant, y compris sous une forme hydratée ou solvatée, comprenant la réaction d'un composé de formule (3), ou d'un sel d'addition d'acide en dérivant, 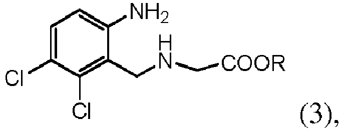 dans lequel R est un groupe alkyle en C₁-C₄, avec du chlorhydrate de chloroformamidine de formule (8), 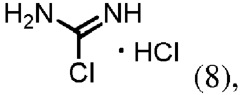 dans un solvant inerte, suivie par le traitement du mélange réactionnel par une base.

2. Le procédé selon la revendication 1, dans lequel R est un groupe éthyle.

3. Le procédé selon la revendication 1 ou 2, dans lequel le solvant inerte est choisi dans le groupe consistant en hydrocarbures aliphatiques en C₅ à C₁₀, hydrocarbures alicycliques en C₅-C₁₀, hydrocarbures aromatiques en C₆ à C₁₀, hydrocarbures chlorés en C₁-C₆, éthers aliphatiques ou cycliques en C₅-C₁₀, esters aliphatiques en C₃ à C₁₀, diméthylformamide, diméthylsulfoxyde, acétonitrile, acide acétique ou toute combinaison de ceux-ci.

4. Le procédé selon l'une quelconque des revendications 1-3, dans lequel la température de réaction est comprise entre 50°C et la température de reflux.

5. Le procédé selon l'une quelconque des revendications 1-4, dans lequel le produit de la réaction entre les composés de formules (3) et (8) est isolé du mélange réactionnel avant le traitement par la base.

6. Le procédé selon l'une quelconque des revendications 1-5, dans lequel la base est une amine organique ou un carbonate métallique.

7. Le procédé selon l'une quelconque des revendications 1-6, dans lequel l'anagrélide est isolé du mélange réactionnel sous forme de base libre après le traitement par la base.

8. Le procédé selon la revendication 7, dans lequel l'anagrélide sous forme de base libre est convertie en chlorhydrate d'anagrélide, et est isolé sous la forme d'une matière solide, éventuellement sous une forme solvatée ou hydratée.

9. Composé de formule (9a) ou (9b), ou un sel d'addition d'acide en dérivant, 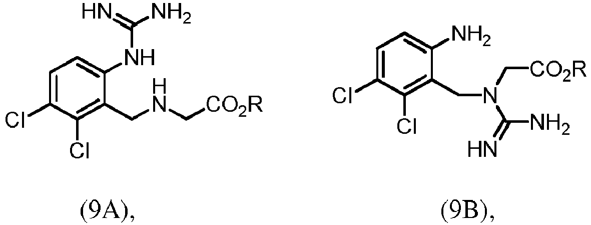 dans lequel R est un groupe alkyle en C₁-C₄, de préférence un groupe éthyle ou un mélange de ceux-ci.
